# EUROPÄISCHE PATENTSCHRIFT

(11) **EP 1 153 909 B1**
(45) Veröffentlichungstag und Bekanntmachung des Hinweises auf die Patenterteilung: **13.10.2004**
(21) Anmeldenummer: 01110519.4
(22) Anmeldetag: 28.04.2001
(51) Int. Cl.: C07C 47/55, C07C 45/42, C07C 45/68, C07C 45/00

(54) **Ortho-substituierte Benzaldehyde, Verfahren zu ihrer Herstellung und ihre Verwendung**
Ortho-substituted benzaldehydes, process for their preparation, and their use
Benzaldéhydes ortho-substitués, procédé pour leur préparation, et leur emploi

(30) Priorität: 10.05.2000 DE 10022661
(43) Veröffentlichungstag der Anmeldung: 14.11.2001
(73) Patentinhaber: Clariant GmbH, 65929 Frankfurt am Main (DE)
(72) Erfinder: Wingen, Rainer, Dr., 65795 Hattersheim (DE); Schmidt, Wolfgang, Dr., 63303 Dreieich (DE)
(74) Vertreter: Isenbruck, Günter

(56) Entgegenhaltungen:
- EP-A- 0 211 584
- EP-A- 0 568 289
- WO-A-01/68653
- WO-A-98/14433

## Beschreibung

Benzaldehyde mit ortho-Halogen-Substituenten bzw. mit insgesamt bis zu fünf Halogen-Substituenten sind vielfältig in der chemischen Literatur als Ausgangsmaterialien beschrieben für die Synthese von Wirkstoffen (z.B. EP-A- 0 174 131, EP-A- 0 271 240, EP-A- 0 289 942, FR-A- 1 514 517, US-A- 3 856 504, US-A- 3 982 020, US-A- 4 902 814) oder allgemein als Intermediat (DE-A 3310953). Aldehyde, die in der einen ortho-Position einen Chlor-, Brom- oder Iod-Substituenten sowie in der anderen ortho-Position einen Fluor-Substituenten tragen, sind in Form von 2-Chlar-6-fluorbenzaldehyd, 2-Brom-6-fluorbenzaldehyd und 2-Fluor-6-iodbenzaldehyd bekannt. Es besteht aber ein Bedarf an Verbindungen mit mehreren Fluor-Substituenten und zusätzlichen Funktionalitäten, da über eine Fluorierung bzw. einen unterschiedlichen Fluorierungsgrad sehr oft die Aktivität von Agrochemikalien oder Pharmazeutika gesteigert wird durch Veränderung der Lipophilie und/oder des Dipolmomentes (Kanie et al., Bull. Chem. Soc. Jpn. 2000, 73, 471).

WO 01/68653 betrifft Dihydroimidazo[2,1-B]thiazol und Dihydro-5H-thiazolo[3,2-A]pyrimidine als Antidepressiva.

Beispiel 38 offenbart unter anderem die Herstellung von 2-Brom-5,6-difluorbenzaldehyd ausgehend von 4-Brom-1,2-difluorbenzol durch Lithiierung mit Lithiumdiisopropylamid und nachfolgende Umsetzung mit Dimethylformamid bei einer Temperatur von -75 °C.

WO 98/14433 betrifft Aralkyl- und Aralkyliden-heterocyclische Lactame und Imide. Gemäß Herstellung 2 wird 2,4-Dibrom-6-fluorbenzaldehyd hergestellt.

EP-A-0 568 289 betrifft Benzothiophene und Thienothiophene und verwandte Verbindungen, die beispielsweise als Urokinase-Inhibitoren eingesetzt werden können. Unter anderem wird die Formylierung von 2-Chlor-4-fluoriodbenzol mit LDA und DMF beschrieben.

EP-A-0 211 584 betrifft Antihypertensiv-Mittel. Gemäß Beispiel 24 (a) wird 2,3-Dichlor-6-fluorbenzaldehyd analog zur Herstellung von 2,3-Difluorbenzaldehyd hergestellt.

Benzaldehyde mit mehreren Fluor-Substituenten, einer davon in ortho-Stellung zur Aldehyd-Funktion, sowie einem ortho-Halogen-Substituenten (der nicht gleich Fluor ist), obwohl zum Teil durch generische Formulierungen in oben genannten Literaturstellen konstruierbar, sind bis auf 2-Brom-5,6-difluorbenzaldehyd nicht bekannt.

Gegenstand der Erfindung sind daher ortho-substituierte Benzaldehyde der Formel (I) 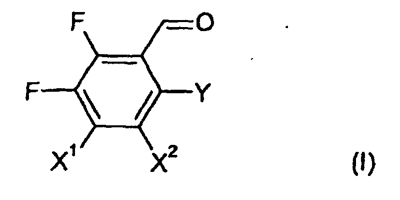 worin die Substituenten X¹, X² und Y folgende Bedeutung haben:
X¹ = H oder F
X² = H oder F
Y = Cl, Br oder I
ausgenommen X¹, X² H und Y Br.

Bevorzugt werden Verbindungen in denen
a) X¹ und X²: H
   Y: Cl, oder
b) X¹ : F X²: H
   Y: Cl oder Br ; oder
c) X¹ und X²: F
   Y: Cl oder Br
bedeuten;
insbesondere
2-Chlor-5,6-difluorbenzaldehyd
2-Chlor-4,5,6-trifluorbenzaldehyd
2-Brom-4,5,6-trifluorbenzaldehyd

Ein weiterer Gegenstand der Erfindung ist ein Verfahren zur Herstellung der Verbindungen der Formel (I), dadurch gekennzeichnet, dass ein Halogenbenzol der Formel (II) - in dem Y, X¹ und X² die Bedeutung wie in (I) besitzen - in einem Lösemittel oder Lösemittelgemisch bei einer Temperatur, die der Arinbildung nicht Vorschub leistet, mit einer organischen Lithiumverbindung umgesetzt wird. Das Molverhältnis Lithiumverbindung : Ausgangsprodukt (II) beträgt vorzugsweise 1:1 bis 1,2:1. Die erzeugte Lithiumverbindung wird, wiederum bei einer Temperatur, die der Arinbildung nicht Vorschub leistet, mit einem Formyläquivalent der Formel (III) umgesetzt und anschließend einer Hydrolyse zu (I) unterworfen. Das Molverhältnis (II): (III) beträgt vorzugsweise 1:1 bis 1:2. 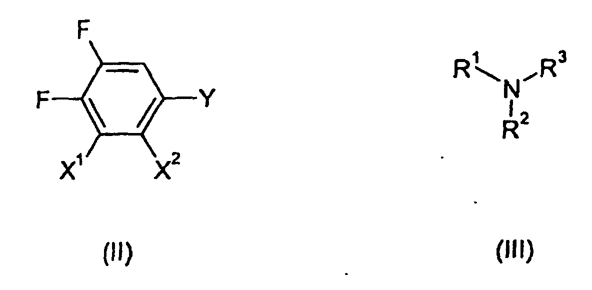 Hierbei bedeuten in (III) R¹ einen Alkylrest mit 1 bis 6 C-Atomen, einen Trimethylsilylrest oder (gegebenenfalls substituiertes) Phenyl, R² einen Alkylrest von 1 bis 6 C-Atomen, einen Trimethylsilylrest oder (gegebenenfalls substituiertes) Phenyl, R³ -CH(=O) oder -CH(OR⁴)₂; R¹ und R² können auch zusammen mit dem Stickstoffatom Teil eines fünf- bis siebengliedrigen Ringes sein. R⁴ bedeutet einen Alkylrest von 1 bis 4 C-Atomen.

Die Erfindung betrifft zudem ein Verfahren zur Herstellung von 2-Brom-5,6-difluorbenzaldehyd, bei dem 4-Brom-1,2-difluorbenzol in einem Lösemittel oder Lösemittelgemisch bei einer Temperatur unterhalb - 60 °C mit einer organischen Lithiumverbindung, ausgewählt aus Lithium-2,2,6,6-tetramethylpiperidid, Lithiumdicyclohexylamid, Lithiumcyclohexylisopropylamid oder Lithiumbis(trimethylsilyl)-amid, und anschließend mit einem Formyläquivalent der Formel (III) umgesetzt wird.

Bevorzugt erfolgt die Umsetzung von (II) mit der organischen Lithiumverbindung bei einer Temperatur unterhalb von -60°C, ganz besonders bevorzugt unterhalb von -70°C, insbesondere bei einer Temperatur im Bereich von -70°C bis -110°C. Die Reaktionszeiten betragen in der Regel 1 bis 8 Stunden. Nach Abschluss der Reaktion (z.B. durch DC oder GC detektierbar) wird die Reaktionsmischung langsam bis auf -25 bis -15 °C erwärmt und vorsichtig mit Wasser hydrolysiert. Danach wird mit Salzsäure bis auf pH 1 bis 5 angesäuert und mit einem geeigneten Lösemittel (z.B. tert.-Butylmethylether, Dichlormethan, Ethylacetat, Toluol) extrahiert. Die Extrakte der organischen Phase werden vereinigt und beispielsweise mit Natriumsulfat getrocknet. Das Lösemittel kann im Vakuum abgetrennt und so der gewünschte ortho-substituierte Benzaldehyd der Formel (III) erhalten werden. Eine gegebenenfalls erforderliche Reinigung kann erfolgen durch Chromatographie, Destillation oder Kristallisation oder einer Kombination der genannten Methoden. Die Ausbeuten liegen üblicherweise im Bereich von 50 bis 80 %, bezogen auf (II). Die organische Lithiumverbindung ist bevorzugt die Lithiumverbindung eines sekundären Amins, vorzugsweise mit sterisch anspruchvollen Substituenten. Besonders bevorzugt ist Lithiumdiisopropylamid, Lithium-2,2,6,6-tetramethylpiperidid, Lithiumdicyclohexylamid, Lithiumcyclohexylisopropylamid oder Lithiumbis-(trimethylsilyl)-amid. Ganz besonders bevorzugt ist Lithium-2,2,6,6-tetramethylpiperidid. Für die Verbindungen der Formel (II), in denen Y Cl bedeutet, ist die organische Lithiumverbindung eine Alkyllithiumverbindung oder die Lithiumverbindung eines sekundären Amins; bevorzugt ist n-Butyllithium. Gegebenenfalls kann auch Kalium-tertbutylat zur besseren Aktivierung zugesetzt werden.

In einer bevorzugten Ausführungsform bedeutet Y in Formel (II) Br.
Es kann auch vorteilhaft sein, dem Reaktionsgemisch aktivierende bzw. die Selektivität beeinflussende Stoffe zuzusetzen, z.B. Tetramethylethylendiamin oder Kälium-tertbutylat (letzteres bevorzugt im Fall der Verbindungen der Formel (II), in denen Y Cl bedeutet).

Bevorzugt ist das Formyläquivalent der Formel (III) N,N-Dimethylformamid, N,N-Diethylformamid, N,N-Dipropylformamid, N,N-Diisopropylformamid, N,N-Dibutylformamid, N-Formylpyrrolidin, N-Formylmorpholin, N-Formylpiperidin, Dimethylformamid-dialkylacetal, N-Methylformanilid, N-Ethylformanilid öder N,N-Bis-(trimethylsilyl)-formamid.
Ganz besonders bevorzugt ist das Formyläquivalent der Formel (III) N,N-Dimethylformamid.

Als Lösemittel im Sinne der Erfindung sind aprotische Lösemittel geeignet, beispielsweise Ether wie Tetrahydrofuran, 1,2-Dimethoxyethan, 1,4-Dioxan, Diethylether, oder Kohlenwasserstoffe wie Hexan, Cyclohexan, Heptan, Pentan, oder Mischungen aprotischer Lösemittel.

Die Erfindung betrifft ferner ein Verfahren zur Herstellung von Verbindungen der Formel (I) 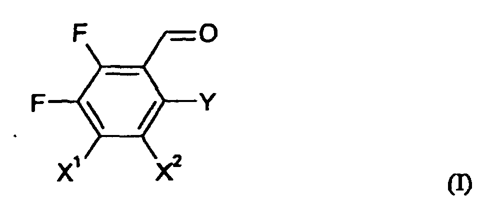 worin X¹, X² und Y folgende Bedeutung haben:
- X¹: H oder F
- X²: H oder F
- Y: Cl, Br oder I,
bei dem ein Halogenbenzol der Formel (II) - in dem Y, X1 und X2 die gleiche Bedeutung wie in Formel I haben - in einem Lösemittel oder Lösemittelgemisch mit einer organischen Lithiumverbindung in Gegenwart eines Formyläquivalents der Formel (III) umgesetzt wird 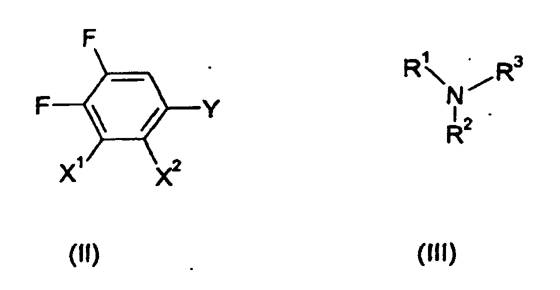 und anschließend hydrolysiert wird, wobei in Formel (III) R¹ einen Alkylrest mit 1 bis 6 C-Atomen, einen Trimethylsilylrest oder (gegebenenfalls substituiertes) Phenyl bedeutet, R² einen Alkylrest von 1 bis 6 C-Atomen, einen Trimethylsilylrest oder (gegebenenfalls substituiertes) Phenyl bedeutet und R³ für -CH(=O) oder -CH(OR⁴)₂ steht, wobei R¹ und R² gegebenenfalls zusammen mit dem Stickstoffatom Teil eines fünf- bis siebengliedrigen Ringes sein können und R⁴ eine Alkylrest von 1 bis 4 C-Atomen bedeutet.

Die Umsetzung von (II) mit der organischen Lithiumverbindung kann damit auch in Gegenwart der Verbindung (III) erfolgen, so dass in situ gebildetes Lithiumderivat aus (II) direkt mit (III) reagieren kann. Hierzu kann es erforderlich und vorteilhaft sein, die Reaktion oberhalb von -60°C, z.B. im Bereich von -20 bis +25°C, durchzuführen.

Ein weiterer Gegenstand der Erfindung ist die Verwendung von Verbindungen der Formel (I) mit Ausnahme von 2-Brom -5,6-difluorbenzaldehyd als Ausgangsmaterialien für die Herstellung von Agrochemikalien, Elektronikmaterialien - insbesondere Flüssigkristallen - und Pharmazeutika. Hierzu sind die Verbindungen der Formel (I) in besonderem Maße geeignet, da sowohl die Aldehyd-Funktion (z.B. über Wittig-Reaktion, Reduktion zum Benzylalkohol, Kondensation mit C-H-, N-H- oder S-H-Verbindungen) als auch die Halogen-Funktion (worunter hier die Funktion des Substituenten Y verstanden werden soll) (z.B. über Suzuki-Kupplung, Grignard-Reaktion, Heck-Reaktion) für Reaktionen zur Verfügung stehen sowie auch - unter speziellen Bedingungen im Falle der Verbindungen bzw. der daraus abgeleiteten Folgeprodukte, bei denen X¹ F bedeutet, ganz besonders im Falle der Verbindungen bzw. der daraus abgeleiteten Folgeprodukte, bei denen X¹ und X² F bedeuten - die Reaktivität eines Difluor-Aromaten (z.B. über ortho-Metallierung) bzw. Trifluoraromaten (z. B. über ortho-Metallierung oder aromatische nucleophile Substitution) bzw. Tetrafluoraromaten (z.B. über aromatische nucleophile Substitution) ausgenutzt werden kann.

### Beispiele:

### Beispiel 1 2-Brom-5,6-difluorbenzaldehyd

Zu einer Lösung von 152 mmol 2,2,6,6-Tetramethylpiperidin und 152 mmol n-Butyllithium (1,6 M Lösung in n-Hexan) in 280 ml trockenem Tetrahydrofuran werden bei -75°C 145 mmol 1-Brom-3,4-difluorbenzol gegeben. Man belässt 4 Stunden bei dieser Temperatur und versetzt dann tropfenweise mit 174 mmol DMF. Anschließend lässt man die Reaktionsmischung langsam auftauen, hydrolysiert bei -20°C mit Wasser, säuert mit Salzsäure an und extrahiert mit tert.-Butylmethylether. Die vereinigten org. Extrakte werden mit ges. Natriumchloridlösung gewaschen und mit Natriumsulfat getrocknet. Nach dem Entfernen der Lösemittel im Vakuum wird das Rohprodukt durch Chromatographie an Kieselgel (Eluent: Dichlormethan/n-Heptan 1:1) und Umkristallisation aus n-Heptan gereinigt. Man erhält 18 g (56 %) 2-Brom-5,6-difluorbenzaldehyd in Form leicht gelblicher Kristalle. - Schmp.: 40-43,5°C.- ¹H-NMR (400 MHz, CDCl₃/TMS): δ = 10,31 (dd, ⁴*J*_{(HF)} = 2 Hz, ⁵*J*_{(HF)} = 1,5 Hz; 1 H, CHO), 7,45 (ddd, ³*J*_{(HH)} = 9 Hz, ⁴*J*_{(HF)} = 4 Hz, ⁵*J*_{(HF)} = 2 Hz, 1 H, Hₐᵣ), 7,28 (ddd, ³*J*_{(HH)} = 9 Hz, ³*J*_{(HF)} = 9 Hz, ⁴*J*_{(HF)} = 8 Hz, 1 H, Hₐᵣ).- ¹⁹F-NMR (376,5 MHz, ¹H-breitband-entkoppelt, CDCl₃/CFCl₃): δ = -136,4 (d, ³*J* = 19,2 Hz), -139,2 (d, ³*J* = 19,2 Hz).

### Beispiel 2

2-Brom-4,5,6-trifluorbenzaldehyd kann analog Beispiel 1 aus 1-Brom-3,4,5-trifluorbenzol erhalten werden. - ¹H-NMR (400 MHz, CDCl₃/TMS): δ = 10,24 (dd, ⁴*J*_{(HF)} = 1,5 Hz, ⁵*J*_{(HF)} = 1 Hz, 1 H, CHO), 7,38 (ddd, ³*J*_{(HH)} = 9 Hz, ⁴*J*_{(HF)} = 6 Hz, ⁵*J*_{(HF)} = 2 Hz, 1 H, Hₐᵣ).- ¹⁹F-NMR (376,5 MHz, ¹H-breitband-entkoppelt, CDCl₃/CFCl₃): δ = -123,4 (dd, ³*J* = 20 Hz und 13 Hz), -135,9 (dd, ³*J* = 19 Hz und 13 Hz), -158,0 (dd, ³*J* = 20 Hz und 19 Hz).

### Beispiel 3

2-Chlor-5,6-difluorbenzaldehyd kann analog Beispiel 1 aus 1-Chlor-3,4-difluorbenzol erhalten werden.

### Beispiel 4

2-Brom-3,4,5,6-tetrafluorbenzaldehyd kann analog Beispiel 1 aus 1-Brom-2,3,4,5-tetrafluorbenzol erhalten werden.

### Beispiel 5

2-Chlor-3,4,5,6-tetrafluorbenzaldehyd kann analog Beispiel 1 aus 1-Chlor-2,3,4,5-tetrafluorbenzol erhalten werden.

### Beispiel 6

2-lod-5,6-difluorbenzaldehyd kann analog Beispiel 1 aus 3,4-Difluoriodbenzol erhalten werden.

### Anwendungsbeispiel 1

Analog Kumar, J. Org. Chem. 1997, 62, 8535-8539 wird 2-Brom-5,6-difluorbenzaldehyd mit 4-Pentylphenylboronsäure unter Pd-Katalyse zu 3,4-Difluor-4'-pentylbiphenyl-2-carbaldehyd umgesetzt. Dieses wird analog Kumar, loc. cit., mit Trimethylsulfoniumiodid zu 3,4-Difluor-2-(oxiran-2-yl)-4'-pentyl-biphenyl umgesetzt, das ohne weitere Reinigung in Gegenwart von BF₃-Etherat zu 1,2-Difluor-7-pentylphenanthren umgesetzt wird. Die Reinigung des Rohprodukts erfolgt nach der für diesen Typ Flüssigkristallmaterialien typischen Prozedur (vgl. DE-A 195 00 768). Man erhält 1,2-Difluor-7-pentylphenanthren in Form farbloser Kristalle.

## Patentansprüche

1. Verbindungen der Formel (I) 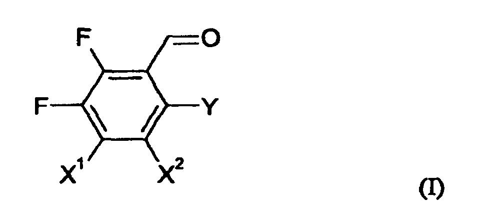 worin X¹, X² und Y folgende Bedeutung haben:
X¹ H oder F
X² H oder F
Y Cl, Br oder I,
ausgenommen X¹, X²H und Y Br.

2. Verbindungen nach Anspruch 1, worin
a) X¹ und X²: H
Y: Cl
b) X¹: F X²: H
Y: Cl oder Br
c) X¹ und X²: F
Y: Cl oder Br
bedeuten.

3. Verfahren zur Herstellung der Verbindungen gemäß Anspruch 1 oder 2, **dadurch gekennzeichnet, dass** ein Halogenbenzol der Formel (II) - in dem Y, X¹ und X² die gleiche Bedeutung wie in Anspruch 1 oder 2 haben - in einem Lösemittel oder Lösemittelgemisch bei einer Temperatur unterhalb - 60 °C mit einer organischen Lithiumverbindung und anschließend mit einem Formyläquivalent der Formel (III) umgesetzt wird 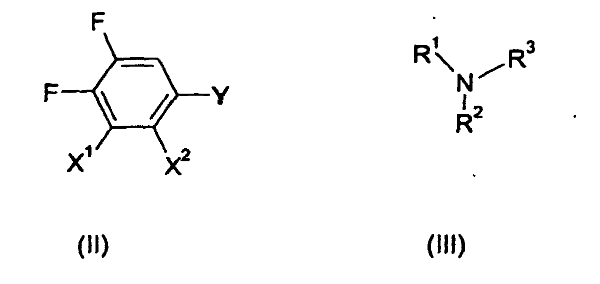 und anschließend hydrolysiert wird, wobei in Formel (III) R¹ einen Alkylrest mit 1 bis 6 C-Atomen, einen Trimethylsilylrest oder (gegebenenfalls substituiertes) Phenyl bedeutet, R² einen Alkylrest von 1 bis 6 C-Atomen, einen Trimethylsilylrest oder (gegebenenfalls substituiertes) Phenyl bedeutet und R³ für -CH(=O) oder -CH(OR⁴)₂ steht, wobei R¹ und R² gegebenenfalls zusammen mit dem Stickstoffatom Teil eines fünf- bis siebengliedrigen Ringes sein können und R⁴ einen Alkylrest von 1 bis 6 C-Atomen, bedeutet.

4. Verfahren nach Anspruch 3, **dadurch gekennzeichnet, dass** die organische Lithiumverbindung Lithium-2,2,6,6-tetramethylpiperidid oder Lithiumdiisopropylamid ist oder, im Falle Y = Cl, Alkyllithium ist.

5. Verfahren zur Herstellung von 2-Brom-5,6-difluorbenzaldehyd, **dadurch gekennzeichnet, dass** 4-Brom-1,2-difluorbenzol in einem Lösemittel oder Lösemittelgemisch bei einer Temperatur unterhalb -60 °C mit einer organischen Lithiumverbindung, ausgewählt aus Lithium-2,2,6,6-tetramethylpiperidid, Lithiumdicyclohexylamid, Lithiumcyclohexylisopropylamid oder Lithiumbistrimethylsilyl)-amid, und anschließend mit einem Formyläquivalent der Formel (III) umgesetzt wird 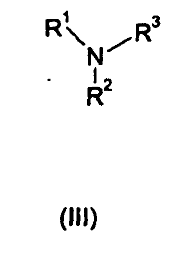 und anschließend hydrolisiert wird, wobei in Formel (III) R' einen Alkylrest mit 1 bis 6 C-Atomen, einen Trimethylsilylrest oder (gegebenenfalls substituiertes) Phenyl bedeutet, R² einen Alkylrest von 1 bis 6 C-Atomen, einen Trimethylsilylrest oder (gegebenenfalls substituiertes) Phenyl bedeutet und R³ für -CH(=O) oder -CH(OR⁴)₂ steht, wobei R¹ und R² gegebenenfalls zusammen mit dem Stickstoffatom Teil eines fünf- bis siebengliedrigen Ringes sein können und R⁴ einen Alkylrest von 1 bis 4 C-Atomen bedeutet.

6. Verfahren nach einem der Ansprüche 3 bis 5, **dadurch gekennzeichnet, dass** die Temperatur unterhalb von -70 °C liegt.

7. Verfahren nach einem der Ansprüche 3 bis 6, **dadurch gekennzeichnet, dass** die Verbindung der Formel (III) Dimethylformamid oder Diethylformamid ist.

8. Verfahren nach einem der Ansprüche 3 bis 7, **dadurch gekennzeichnet, dass** dem Reaktionsgemisch als aktivierende oder die Selektivität beeinflussende Stoffe Tetramethylendiamin oder Kaliumtertbutylat zugesetzt werden.

9. Verfahren zur Herstellung von Verbindungen der Formel (I) 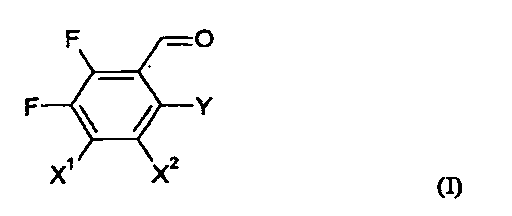 worin X¹, X² und Y folgende Bedeutung haben:
X¹ H oder F
X² H oder F
Y Cl, Br oder I,
**dadurch gekennzeichnet, dass** ein Halogenbenzol der Formel (II) - in dem Y, X¹ und X² die gleiche Bedeutung wie in Formel I haben - in einem Lösemittel oder Lösemittelgemisch mit einer organischen Lithiumverbindung in Gegenwart eines Formyläquivalents der Formel (III) umgesetzt wird 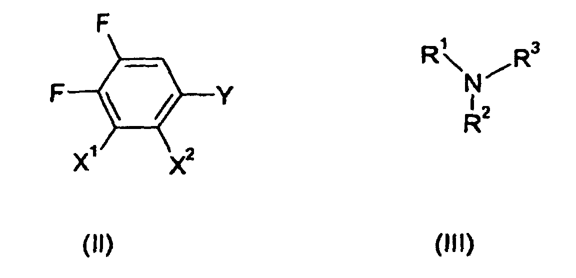 und anschließend hydrolysiert wird, wobei in Formel (III) R¹ einen Alkylrest mit 1 bis 6 C-Atomen, einen Trimethylsilylrest oder (gegebenenfalls substituiertes) Phenyl bedeutet, R² einen Alkylrest von 1 bis 6 C-Atomen, einen Trimethylsilylrest oder (gegebenenfalls substituiertes) Phenyl bedeutet und R³ für -CH(=O) oder -CH(OR⁴)₂ steht, wobei R¹ und R² gegebenenfalls zusammen mit dem Stickstoffatom Teil eines fünf- bis siebengliedrigen Ringes sein können und R⁴ einen Alkylrest von 1 bis 4 C-Atomen bedeutet.

10. Verfahren nach Anspruch 9, **dadurch gekennzeichnet, dass** die Reaktion bei einer Temperatur im Bereich von -20 bis 25 °C durchgeführt wird.

11. Verwendung der Verbindungen nach Anspruch 1 oder 2 als Ausgangsmaterial für die Herstellung von Agrochemikalien, Elektronikmaterialien und Pharmazeutika.

12. Verwendung von 2-Brom-5,6-difluorbenzaldehyd als Ausgangsmaterial für die Herstellung von Elektronikmaterialien.

13. Verwendung nach Anspruch 11 oder 12, **dadurch gekennzeichnet, dass** die Elektronikmaterialien Komponenten flüssigkristalliner Mischungen sind.

14. Verwendung nach Anspruch 13, **dadurch gekennzeichnet, dass** die Komponenten flüssigkristalliner Mischungen Phenanthrenderivate sind.

## Claims

1. A compound of the formula (I) 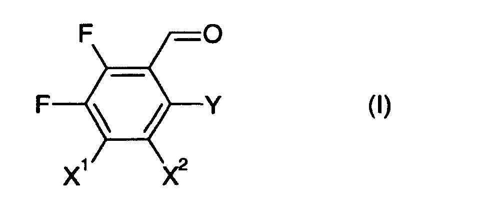 in which X¹, X² and Y have the following meanings:
X¹ is H or F
X² is H or F
Y is Cl, Br or I,
with the exception X¹, X² H and Y Br.

2. The compound as claimed in claim 1, wherein
a) X¹ and X² are H
Y is Cl
b) X¹ is F, X² is H
Y is Cl or Br
c) X¹ and X² are F
Y is Cl or Br.

3. A process for preparing a compound as claimed in claim 1 or 2, which comprises reacting, in a solvent or solvent mixture at a temperature below -60°C, a halobenzene of the formula (II), in which Y, X¹ and X² are as defined in claim 1 or 2, with an organic lithium compound and then with a formyl equivalent of the formula (III) 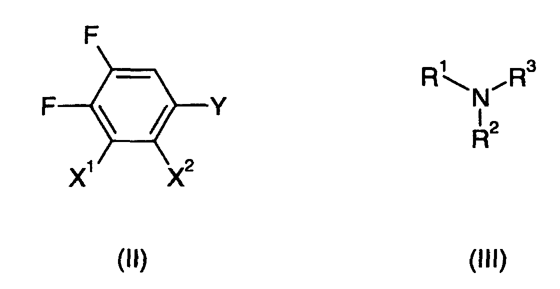 followed by hydrolysis, where in formula (III) R¹ is an alkyl radical having 1 to 6 carbon atoms, a trimethylsilyl radical or (substituted or unsubstituted) phenyl, R² is an alkyl radical having 1 to 6 carbon atoms, a trimethylsilyl radical or (substituted or unsubstituted) phenyl, and R³ is -CH(=O) or -CH(OR⁴)₂, where R¹ and R², together with the nitrogen atom, may also be part of a five- to seven-membered ring and R⁴ is an alkyl radical having 1 to 4 carbon atoms.

4. The process as claimed in claim 3, wherein said organic lithium compound is lithium 2,2,6,6-tetramethylpiperidide or lithium diisopropylamide or, when Y = Cl, is alkyllithium.

5. A process for preparing 2-bromo-5,6-difluorobenzaldehyde which comprises reacting, in a solvent or solvent mixture at a temperature below -60°C, 4-bromo-1,2-difluorobenzene with an organic lithium compound selected from lithium-2,2,6,6-tetramethylpiperidide, lithiumdicyclohexylamide, lithiumcyclohexylisopropylamide or lithium-bis(trimethylsilyl)amide, and then with a formyl equivalent of the formula (III) 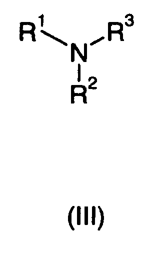 followed by hydrolysis, where in formula (III) R¹ is an alkyl radical having 1 to 6 carbon atoms, a trimethylsilyl radical or (substituted or unsubstituted) phenyl, R² is an alkyl radical having 1 to 6 carbon atoms, a trimethylsilyl radical or (substituted or unsubstituted) phenyl, and R³ is -CH(=O) or -CH(OR⁴)₂, where R¹ and R², together with the nitrogen atom, may also be part of a five- to seven-membered ring and R⁴ is an alkyl radical having 1 to 4 carbon atoms.

6. The process as claimed in one of claims 3 to 5, wherein the temperature is below -70°C.

7. The process as claimed in one of claims 3 to 6, wherein said compound of the formula (III) is dimethylformamide or diethylformamide.

8. The process as claimed in one of claims 3 to 7, wherein tetramethylenediamine or potassium tert-butylate, are added to the reaction mixture as activators or selectivity modifiers.

9. A process for preparing compounds of the formula (I) 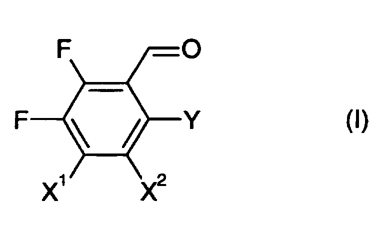 in which X¹, X² and Y have the following meanings:
X¹ is H or F
X² is H or F
Y is Cl, Br or I,
which comprises reacting, in a solvent or solvent mixture, a halobenzene of the formula (II), in which Y, X¹ and X² are as defined in formula (I), with an organic lithium compound in the presence of a formyl equivalent of the formula (III) 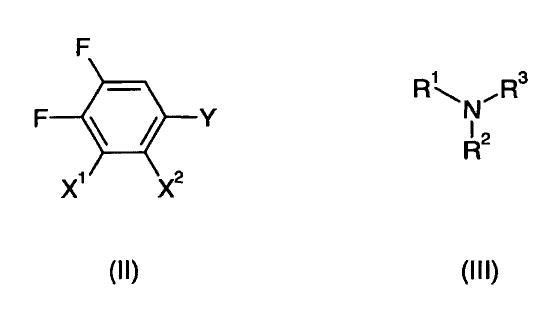 followed by hydrolysis, where in formula (III) R¹ is an alkyl radical having 1 to 6 carbon atoms, a trimethylsilyl radical or (substituted or unsubstituted) phenyl, R² is an alkyl radical having 1 to 6 carbon atoms, a trimethylsilyl radical or (substituted or unsubstituted) phenyl, and R³ is -CH(=O) or -CH(OR⁴)₂, where R¹ and R², together with the nitrogen atom, may also be part of a five- to seven-membered ring and R⁴ is an alkyl radical having 1 to 4 carbon atoms.

10. The process as claimed in claim 9, wherein the reaction is carried out at a temperature in the range from -20 to +25°C.

11. The use of a compound as claimed in claim 1 or 2 as starting material for preparing agrochemicals, electronic materials and pharmaceuticals.

12. The use of 2-bromo-5,6-difluorobenzaldehyde as starting material for the manufacture of electronic materials.

13. The use as claimed in claim 11 or 12, wherein said electronic materials are components of liquid-crystalline mixtures.

14. The use as claimed in claim 13, wherein said components of liquid-crystalline mixtures are phenanthrene derivatives.

## Revendications

1. Composés de formule (I) 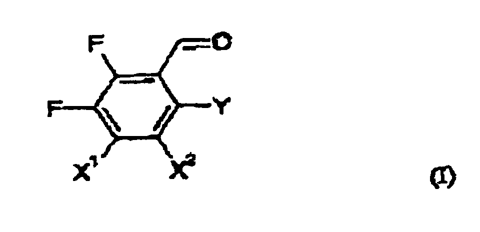 dans laquelle X¹, X² et Y ont la signification suivante :
X¹ désigne H ou F
X² désigne H ou F
Y désigne Cl, Br ou I
excepté X¹, X² qui désignent H et Y qui désigne Br.

2. Composés selon la revendication 1, dans lesquels :
a) X¹ et X² désignent H
Y désigne Cl
b) X¹ désigne F, X² désigne H
Y désigne Cl ou Br
c) X¹ et X² désignent F
Y désigne Cl ou Br.

3. Procédé pour la préparation de composés selon la revendication 1 ou 2, **caractérisé en ce qu'**un halogénobenzène de formule (II), dans laquelle Y, X¹ et X² ont la même signification que dans la revendication 1 ou 2, est mis à réagir, dans un solvant ou un mélange de solvants, à une température inférieure à -60 °C, avec un composé du lithium organique et ensuite avec un équivalent du formyle de formule (III) : 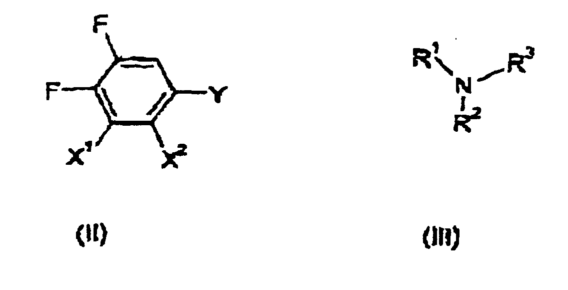 et il est ensuite hydrolysé, dans la formule (III) R¹ désignant un groupe alkyle ayant 1 à 6 atomes de carbone, un groupe triméthylsilyle ou un groupe phényle (éventuellement substitué), R² désignant un groupe alkyle ayant 1 à 6 atomes de carbone, un groupe triméthylsilyle ou un groupe phényle (éventuellement substitué) et R³ étant -CH(=O) ou -CH (OR⁴)₂, R¹ et R² pouvant être éventuellement, conjointement avec l'atome d'azote, une partie d'un cycle ayant cinq à sept chaînons et R⁴ désignant un groupe alkyle ayant 1 à 6 atomes de carbone.

4. Procédé selon la revendication 3, **caractérisé en ce que** le composé du lithium organique est le 2,2,6,6-tétraméthylpipéridure de lithium ou le diisopropylamide de lithium ou, dans le cas où Y = Cl, il est l'alkyl lithium.

5. Procédé pour la préparation de 2-bromo-5,6-difluorobenzaldéhyde, **caractérisé en ce que** du 4-bromo-1,2-difluorobenzène est mis à réagir, dans un solvant ou un mélange de solvants, à une température inférieure à -60 °C, avec un composé du lithium organique, choisi parmi le 2,2,6,6-tétraméthylpipéridure de lithium, le dicyclohexylamide de lithium, le cyclohexylisopropylamide de lithium ou le bis-triméthylsilylamide de lithium, et ensuite avec un équivalent du formyle de formule (III) : 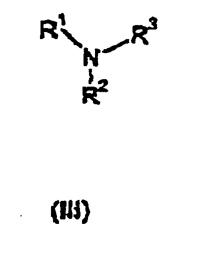 et il est ensuite hydrolysé, dans la formule (III) R¹ désignant un groupe alkyle ayant 1 à 6 atomes de carbone, un groupe triméthylsilyle ou un groupe phényle (éventuellement substitué), R² désignant un groupe alkyle ayant 1 à 6 atomes de carbone, un groupe triméthylsilyle ou un groupe phényle (éventuellement substitué) et R³ étant -CH(=O) ou -CH(OR⁴)₂, R¹ et R² pouvant être éventuellement, conjointement avec l'atome d'azote, une partie d'un cycle ayant cinq à sept chaînons et R⁴ désignant un groupe alkyle ayant 1 à 4 atomes de carbone.

6. Procédé selon l'une des revendications 3 à 5, **caractérisé en ce que** la température se situe au-dessous de -70 °C.

7. Procédé selon l'une des revendications 3 à 6, **caractérisé en ce que** le composé de formule (III) est le diméthylformamide ou le diéthylformamide.

8. Procédé selon l'une des revendications 3 à 7, **caractérisé en ce que** l'on ajoute au mélange réactionnel de la tétraméthylènediamine ou du tert-butylate de potassium en tant que substances activantes ou influençant la sélectivité.

9. Procédé pour la préparation de composés de formule (I) : 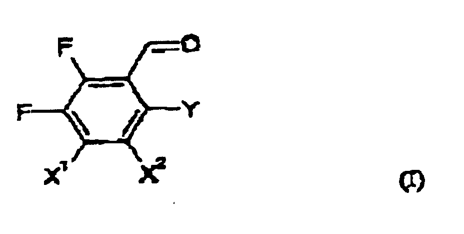 dans laquelle X¹, X² et Y ont la signification suivante :
X¹ désigne H ou F
X² désigne H ou F
Y désigne Cl, Br ou I
**caractérisé en ce qu'**un halogénobenzène de formule (II), dans laquelle Y, X¹ et X² ont la même signification que dans la formule I, est mis à réagir, dans un solvant ou un mélange de solvants,avec un composé du lithium organique en présence d'un équivalent du formyle de formule (III) : 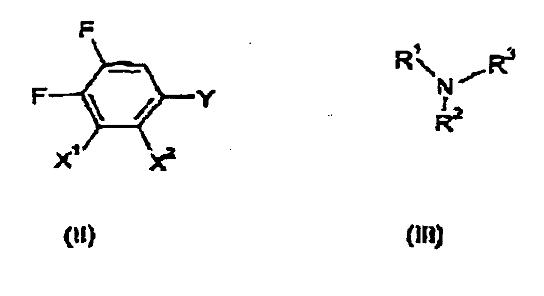 et il est ensuite hydrolysé, dans la formule (III) R¹ désignant un groupe alkyle ayant 1 à 6 atomes de carbone, un groupe triméthylsilyle ou un groupe phényle (éventuellement substitué), R² désignant un groupe alkyle ayant 1 à 6 atomes de carbone, un groupe triméthylsilyle ou un groupe phényle (éventuellement substitué) et R³ étant -CH(=O) ou -CH(OR⁴)₂, R¹ et R² pouvant être éventuellement, conjointement avec l'atome d'azote, une partie d'un cycle ayant cinq à sept chaînons et R⁴ désignant un groupe alkyle ayant 1 à 4 atomes de carbone.

10. Procédé selon la revendication 9, **caractérisé en ce que** la réaction est réalisée à une température située dans la plage allant de -20 à 25 °C.

11. Utilisation des composés selon la revendication 1 ou 2 en tant que produit de départ pour la fabrication de produits agrochimiques, de matériaux électroniques et de produits pharmaceutiques.

12. Utilisation du 2-bromo-5,6-difluorobenzaldéhyde en tant que produit de départ pour la fabrication de matériaux électroniques.

13. Utilisation selon la revendication 11 ou 12, **caractérisée en ce que** les matériaux électroniques sont des composants de mélanges à cristaux liquides.

14. Utilisation selon la revendication 13, **caractérisée en ce que** les composants des mélanges à cristaux liquides sont des dérivés phénanthréniques.
